# EUROPEAN PATENT APPLICATION

(11) **EP 4 588 913 A1**
(43) Date of publication of application: **23.07.2025**
(21) Application number: 23865369.5
(22) Date of filing: 05.09.2023
(51) Int. Cl.: C07C 319/28, C07C 321/14, C08G 18/28, C08G 18/38, C08G 18/72, G02B 1/04

(54) **POLYTHIOL COMPOSITION, POLYMERIZABLE COMPOSITION, RESIN, MOLDED BODY, OPTICAL MATERIAL, AND LENS**

(30) Priority: 14.09.2022 JP 2022146425
(71) Applicant: Mitsui Chemicals, Inc., Tokyo 104-0028 (JP)
(72) Inventor: TOKUNAGA, Koichi, Omuta-shi, Fukuoka 836-8610 (JP); NAKAI, Shinnosuke, Omuta-shi, Fukuoka 836-8610 (JP)
(74) Representative: Mewburn Ellis LLP
(86) International application number: PCT/JP2023/032431
(87) International publication number: WO 2024/058014

(57) **Abstract**

A polythiol composition, including: a polythiol compound (A); and at least one compound selected from the group consisting of a compound represented by Formula (1) and a compound represented by Formula (2), wherein, in a high-performance liquid chromatography measurement, a total peak area of the at least one compound selected from the group consisting of a compound represented by Formula (1) and a compound represented by Formula (2) is 1.00 or less with respect to a total peak area 100 of compounds contained in the polythiol composition.

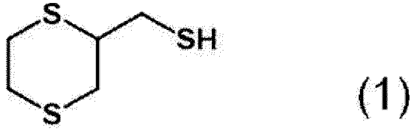

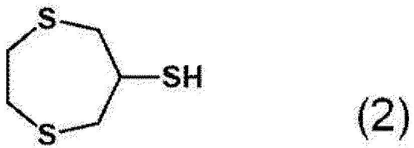

## Description

### TECHNICAL FIELD

The present disclosure relates to a polythiol composition, a polymerizable composition, a resin, a molded body, an optical material, and a lens.

### BACKGROUND ART

Plastic lenses are lighter and less likely to break than inorganic lenses, and can be dyed. Thus, in recent years, plastic lenses have rapidly become widespread in spectacle lenses and optical elements such as camera lenses.

Resins for plastic lenses have been required to have higher performance, and have been required to have a higher refractive index, a higher Abbe number, a lower specific gravity, higher heat resistance, and the like. Various resin materials for lenses have been developed and used so far.

For example, Patent Document 1 describes a mercapto compound represented by a specific structural formula.

For example, Patent Document 2 describes a method of producing a polythiol compound including: a process of reacting 2-mercaptoethanol with an epihalohydrin compound represented by a specific Formula (1) at a temperature of from 10 to 50°C to obtain a polyalcohol compound represented by a specific Formula (2); a process of reacting the obtained polyalcohol compound represented by Formula (2) with thiourea in the presence of hydrogen chloride to obtain an isothiuronium salt; a process of adding ammonia water to a reaction liquid containing the obtained isothiuronium salt within 80 minutes while maintaining the reaction liquid at a temperature of from 15 to 60°C to hydrolyze the isothiuronium salt, thereby obtaining a polythiol compound represented by a specific Formula (5); and a process of adding hydrochloric acid at a concentration of from 25 to 36% to a solution containing the obtained polythiol compound and washing the solution at a temperature of from 10 to 50°C to purify the polythiol compound.
Patent Document 1: Japanese Patent Application Laid-Open (JP-A) No. H02-270859
Patent Document 2: WO 2014-027427A

### SUMMARY OF INVENTION

### Technical Problem

A resin obtained by curing a polymerizable composition containing a polythiol compound may be required to have a reduced yellow index.

Actually, a polymerizable composition containing a polythiol compound often contains a compound other than a polythiol compound.

The present inventors have found that, in the case in which a polymerizable composition contains a compound other than a polythiol compound, the yellow index tends to be increased in the resulting resin.

As a result of various studies on the above tendency, the present inventors have found that it may be difficult to obtain a resin having a reduced yellow index.

An object of an embodiment of the present disclosure is to provide a polythiol composition that enables production of a resin having a reduced yellow index, a polymerizable composition containing the polythiol composition, a resin, a molded body, an optical material, and a lens.

### Solution to Problem

Means to solve the above-described problems include the following embodiments.
<1> A polythiol composition, comprising:
   a polythiol compound (A); and
   at least one compound selected from the group consisting of a compound represented by the following Formula (1) and a compound represented by the following Formula (2): 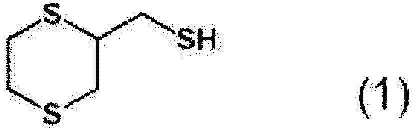 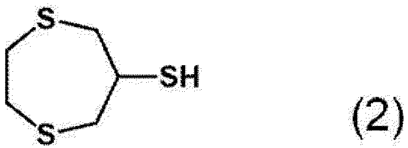
   wherein, in a high-performance liquid chromatography measurement, a total peak area of the at least one compound selected from the group consisting of a compound represented by Formula (1) and a compound represented by Formula (2) is 1.00 or less with respect to a total peak area 100 of compounds contained in the polythiol composition.
<2> The polythiol composition according to <1>, wherein the polythiol compound (A) comprises a polythiol compound obtained from 2-mercaptoethanol and thiourea as raw materials.
<3> The polythiol composition according to <1> or <2>, wherein the polythiol compound (A) comprises 4-mercaptomethyl-1,8-dimercapto-3,6-dithiaoctane or comprises a mixture of 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane.
<4> A polymerizable composition, comprising:
   the polythiol composition according to any one of <1> to <3>; and
   a polyiso(thio)cyanate compound.
<5> The polymerizable composition according to <4>, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.
<6> The polymerizable composition according to <4> or <5>, comprising a polyiso(thio)cyanate composition comprising the polyiso(thio)cyanate compound, wherein the polyiso(thio)cyanate composition comprises: xylylene diisocyanate; and at least one selected from the group consisting of the following compound (N1), the following compound (N2), and the following compound (N3): 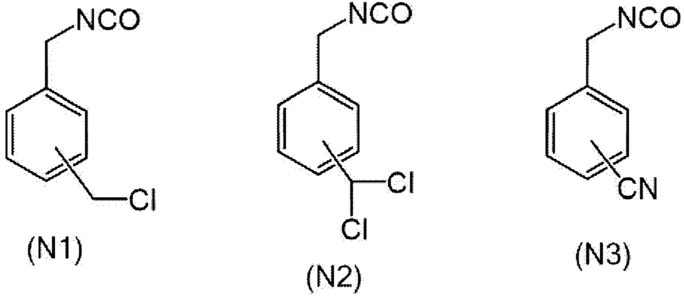 wherein:
   in a case in which the polyiso(thio)cyanate composition comprises the compound (N1), a peak area of the compound (N1) is 0.20 ppm or more with respect to a peak area 100 of xylylene diisocyanate in a high-performance liquid chromatography measurement,
   in a case in which the polyiso(thio)cyanate composition comprises the compound (N2), a peak area of the compound (N2) is 0.05 ppm or more with respect to a peak area 100 of xylylene diisocyanate in a high-performance liquid chromatography measurement, and
   in a case in which the polyiso(thio)cyanate composition comprises the compound (N3), a peak area of the compound (N3) is 0.10 ppm or more with respect to a peak area 100 of xylylene diisocyanate in a high-performance liquid chromatography measurement.
<7> A resin, comprising a cured product of the polymerizable composition according to any one of <4> to <6>.
<8> A molded body, comprising the resin according to <7>.
<9> An optical material, comprising the resin according to <7>.
<10> A lens, comprising the resin according to <7>.

### Advantageous Effects of Invention

According to an embodiment of the present disclosure, a polythiol composition that enables production of a resin having a reduced yellow index, a polymerizable composition containing the polythiol composition, a resin, a molded body, an optical material, and a lens can be provided.

### DESCRIPTION OF EMBODIMENTS

In the present disclosure, a numerical range indicated using "to" indicates a range including numerical values described before and after "to" as a minimum value and a maximum value, respectively.

With regard to the stepwise numerical ranges described herein, the upper limit value or the lower limit value described in a certain numerical range may be replaced with the upper limit value or the lower limit value of another stepwise numerical range or may be replaced with a value indicated in Examples.

Herein, the amount of each component in a material means the total amount of the plurality of substances present in the material, unless otherwise specified, when there is more than one substance corresponding to each component in the material.

Herein, "iso(thio)cyanate " means isocyanate or isothiocyanate.

### <<Polythiol Composition>>

The present disclosure includes the following embodiment A and embodiment B.

That is, a concept of the polythiol composition of the present disclosure encompasses a polythiol composition of embodiment A and a polythiol composition of embodiment B.

### There may be an overlap between embodiment A and embodiment B.

For example, a polythiol composition of embodiment A may have a feature of embodiment B described later.

Herein, the feature of embodiment B, details of which will be described later, is a feature of containing: a polythiol compound (A); and at least one compound selected from the group consisting of a compound represented by Formula (3) described later and a compound represented by Formula (4) described later, wherein, in a high-performance liquid chromatography measurement, a total peak area of the at least one compound selected from the group consisting of a compound represented by Formula (3) and a compound represented by Formula (4) is 2.10 or less with respect to a total peak area 100 of compounds contained in the polythiol composition.

### [Embodiment A]

The polythiol composition of embodiment A contains: a polythiol compound (A); and at least one compound selected from the group consisting of a compound represented by the following Formula (1) and a compound represented by the following Formula (2), wherein, in a high-performance liquid chromatography measurement, a total peak area of the at least one compound selected from the group consisting of a compound represented by Formula (1) and a compound represented by Formula (2) is 1.00 or less with respect to a total peak area 100 of compounds contained in the polythiol composition.

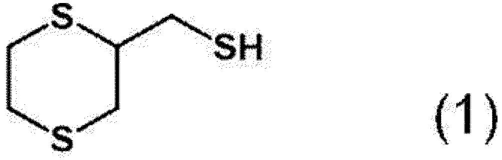

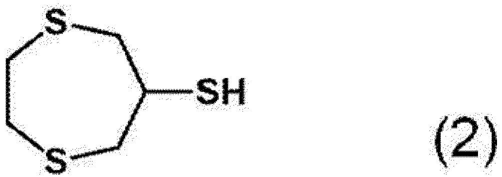

The polythiol composition of embodiment A enables, owing to containing the above-described configuration, production of a resin having a reduced yellow index.

In addition, the polythiol composition of embodiment A also enables production of a resin having a reduced degree of opacity.

### <Polythiol Compound (A)>

The polythiol composition of embodiment A contains a polythiol compound (A).

The polythiol compound (A) of embodiment A is not particularly limited.

The polythiol compound (A) preferably contains a polythiol compound obtained from 2-mercaptoethanol and thiourea as raw materials.

Examples of the polythiol compound obtained from 2-mercaptoethanol and thiourea as raw materials include: at least one selected from the group consisting of 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane; and 4-mercaptomethyl-1,8-dimercapto-3,6-dithiaoctane.

The polythiol compound (A) preferably contains 4-mercaptomethyl-1,8-dimercapto-3,6-dithiaoctane, or at least one selected from the group consisting of 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane, and more preferably contains 4-mercaptomethyl-1,8-dimercapto-3,6-dithiaoctane.

4-Mercaptomethyl-1,8-dimercapto-3,6-dithiaoctane is a compound represented by the following Formula (a-1).

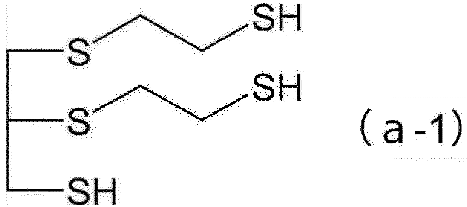

A method of producing the polythiol compound (A) is not particularly limited, and the polythiol compound (A) can be produced by a known method. For example, the polythiol compound (A) can be produced by the method described in WO 2014/027427. The polythiol compound (A) is preferably a compound obtained using a catalyst containing at least one selected from the group consisting of metal hydroxides such as sodium hydroxide and potassium hydroxide, and metal carbonates such as sodium carbonate and potassium carbonate, for example, when reacting 2-mercaptoethanol with an epihalohydrin compound.

The polythiol composition of embodiment A may contain a compound other than the polythiol compound (A) and at least one compound selected from the group consisting of a compound represented by Formula (1) and a compound represented by Formula (2).

For example, the polythiol composition may contain a polythiol compound having a mercapto group or the like other than the polythiol compound (A) and at least one compound selected from the group consisting of a compound represented by Formula (1) and a compound represented by Formula (2) (hereinafter, also referred to as "another polythiol compound").

Examples of another polythiol compound include methanedithiol, 1,2-ethanedithiol, 1,2,3-propanetrithiol, pentaerythritol tetrakis(2-mercaptoacetate), pentaerythritol tetrakis(3-mercaptopropionate), tetrakis(mercaptomethylthiomethyl)methane, tetrakis(2-mercaptoethylthiomethyl)methane, tetrakis(3-mercaptopropylthiomethyl)methane, bis(2-mercaptoethyl)sulfide, bis(2,3-dimercaptopropyl)sulfide, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, 2,5-dimercaptomethyl-1,4-dithiane, 2,5-dimercapto-1,4-dithiane, 2,5-dimercaptomethyl-2,5-dimethyl-1,4-dithiane, 1,1,3,3-tetrakis(mercaptomethylthio)propane, 1,1,2,2-tetrakis(mercaptomethylthio)ethane, and 4,6-bis(mercaptomethylthio)-1,3-dithiane.

### <Compound Represented by Formula (1), Compound Represented by Formula (2)>

The polythiol composition of embodiment A contains at least one compound selected from the group consisting of a compound represented by the following Formula (1) and a compound represented by the following Formula (2).

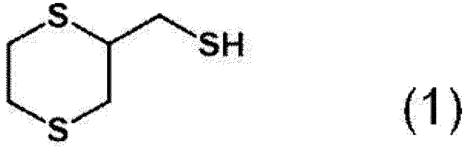

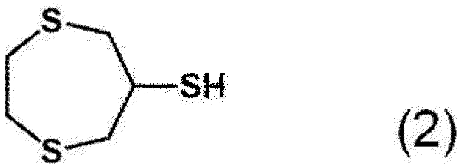

The polythiol composition of embodiment A enables, owing to containing at least one compound selected from the group consisting of a compound represented by Formula (1) and a compound represented by Formula (2), reduction of yellow index and degree of opacity of the resulting resin when the combination of the polythiol compound (A) and the specific sulfur compound is contained in the polymerizable composition.

In the polythiol composition of embodiment A, in a high-performance liquid chromatography measurement, a total peak area of the at least one compound selected from the group consisting of a compound represented by Formula (1) and a compound represented by Formula (2) is 1.00 or less with respect to a total peak area 100 of compounds contained in the polythiol composition.

The total peak area of the at least one compound selected from the group consisting of a compound represented by Formula (1) and a compound represented by Formula (2) being 1.00 or less enables reduction of yellow index and degree of opacity of the resulting resin.

From the same viewpoint as above, the total peak area of the at least one compound selected from the group consisting of a compound represented by Formula (1) and a compound represented by Formula (2) is preferably 0.90 or less, more preferably 0.70 or less, still more preferably 0.50 or less, and particularly preferably 0.30 or less with respect to the total peak area 100 of compounds contained in the polythiol composition.

The lower limit of the total peak area of the at least one compound selected from the group consisting of a compound represented by Formula (1) and a compound represented by Formula (2) with respect to the total peak area 100 of compounds contained in the polythiol composition is not particularly limited, but for example 0.05, and preferably 0.10.

The "total peak area of the at least one compound selected from the group consisting of a compound represented by Formula (1) and a compound represented by Formula (2) with respect to a total peak area 100 of compounds contained in the polythiol composition" means a relative value of the total peak area of the at least one compound selected from the group consisting of a compound represented by Formula (1) and a compound represented by Formula (2) in the case in which the total peak area of the compounds contained in the polythiol composition is taken as 100.

In this regard, for example, when the polythiol composition contains both the compound represented by Formula (1) and the compound represented by Formula (2), the total peak area of the at least one compound selected from the group consisting of a compound represented by Formula (1) and a compound represented by Formula (2) means the total peak area of the compound represented by Formula (1) and the compound represented by Formula (2). When the polythiol composition contains only one of the compound represented by Formula (1) or the compound represented by Formula (2), the total peak area of the at least one compound selected from the group consisting of a compound represented by Formula (1) and a compound represented by Formula (2) means the peak area of the only one of the compounds.

In a high performance liquid chromatography measurement, the total peak area of the at least one compound selected from the group consisting of a compound represented by Formula (1) and a compound represented by Formula (2) is preferably more than 0, more preferably 0.02 or more, still more preferably 0.05 or more, and particularly preferably 0.1 or more, with respect to the total peak area 100 of the compounds contained in the polythiol composition, from the viewpoint of reducing the burden of work of purifying and removing the at least one compound selected from the group consisting of a compound represented by Formula (1) and a compound represented by Formula (2) from the polythiol composition.

In the case in which the total peak area of the at least one compound selected from the group consisting of a compound represented by Formula (1) and a compound represented by Formula (2) satisfies the range of the lower limit, the rate of thickening can be enhanced, and thus polymerization can be facilitated.

A method of adjusting the total peak area of the at least one compound selected from the group consisting of a compound represented by Formula (1) and a compound represented by Formula (2) with respect to the total peak area 100 of the compounds contained in the polythiol composition is not particularly limited, and the total peak area can be adjusted by, for example, operations such as column purification, washing, extraction, or crystallization.

### <Measurement of Peak Area of Compound Represented by Formula (1) and Compound Represented by Formula (2)>

The total peak area of the at least one compound selected from the group consisting of a compound represented by Formula (1) and a compound represented by Formula (2) with respect to the total peak area 100 of the compounds contained in the polythiol composition can be determined by carrying out a high-performance liquid chromatography (HPLC) measurement based on the following conditions.

The peak area appearing at a retention time of 7.5 to 8.0 minutes may be regarded as the peak area of the compound represented by Formula (1) and the compound represented by Formula (2), and the ratio thereof with respect to the total peak area 100 of the compounds contained in the polythiol composition may be calculated.

### (HPLC conditions)

Column: YMC-Pack ODS-AA-312 (S5 Φ 6 mm × 150 mm)
Mobile phase: acetonitrile / 0.01 mol/L-potassium dihydrogen phosphate aqueous solution = 60/40 (vol/vol)
Column temperature: 40°C
Flow rate: 1.0 ml/min
Detector: UV detector, wavelength 230 nm
Preparation of measurement solution: 160 mg of the polythiol composition is dissolved and mixed with 10 ml of acetonitrile
Injection amount: 2 µL

In the HPLC under the above-described conditions, the retention time for the compound represented by Formula (1) and the retention time for the compound represented by Formula (2) are each, for example, 6.0 to 8.0 minutes.

The molecular weight of the compound represented by Formula (1) and the molecular weight of the compound represented by Formula (2) are each 166.

### [Embodiment B]

The polythiol composition of embodiment B contains: a polythiol compound (A); and at least one compound selected from the group consisting of a compound represented by the following Formula (3) and a compound represented by the following Formula (4), wherein, in a high-performance liquid chromatography measurement, a total peak area of the at least one compound selected from the group consisting of a compound represented by Formula (3) and a compound represented by Formula (4) is 2.10 or less with respect to a total peak area 100 of compounds contained in the polythiol composition.

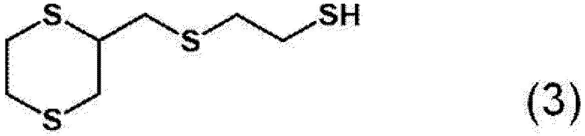

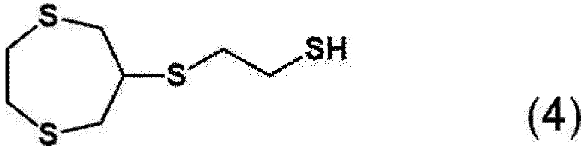

The polythiol composition of embodiment B enables, owing to containing the above-described configuration, production of a resin having a reduced yellow index.

In addition, the polythiol composition of embodiment B also enables production of a resin having a reduced yellow index.

The total peak area of the at least one compound selected from the group consisting of a compound represented by Formula (3) and a compound represented by Formula (4) with respect to the total peak area 100 of the compounds contained in the polythiol composition (embodiment B) can be determined in the same manner as the above-described total peak area of the at least one compound selected from the group consisting of a compound represented by Formula (1) and a compound represented by Formula (2) with respect to the total peak area 100 of the compounds contained in the polythiol composition (embodiment A) (that is, by a HPLC measurement under the same conditions).

The lower limit of the total peak area of the at least one compound selected from the group consisting of a compound represented by Formula (3) and a compound represented by Formula (4) with respect to the total peak area 100 of the compounds contained in the polythiol composition is not particularly limited, but for example 0.10, preferably 0.20, and more preferably 0.30.

In the HPLC under the above-described conditions, the retention time for the compound represented by Formula (3) and the retention time for the compound represented by Formula (4) are each, for example, 9.0 to 11.0 minutes.

The molecular weight of the compound represented by Formula (3) and the molecular weight of the compound represented by Formula (4) are each 226.

### <Polythiol Compound (A)>

The polythiol composition of embodiment B contains a polythiol compound (A).

Details of specific examples, preferable specific examples, preferable aspects, production methods, and the like of the polythiol compound (A) are the same as the details of specific examples, preferable specific examples, preferable aspects, production methods, and the like of the polythiol compound (A) in embodiment A.

The polythiol composition of embodiment B may contain a compound other than the polythiol compound (A) and at least one compound selected from the group consisting of a compound represented by Formula (3) and a compound represented by Formula (4).

For example, the polythiol composition may contain a polythiol compound having a mercapto group or the like other than the polythiol compound (A) and at least one compound selected from the group consisting of a compound represented by Formula (3) and a compound represented by Formula (4) (hereinafter, also referred to as "another polythiol compound").

Details of specific examples and the like of another polythiol compound are the same as the details of specific examples and the like of another polythiol compound in embodiment A.

In this regard, examples of another polythiol compound also include at least one compound selected from the group consisting of a compound represented by Formula (1) and a compound represented by Formula (2), which is a feature of embodiment A.

That is, the polythiol composition of embodiment B may also have the above-described feature of the polythiol composition of embodiment A.

### <Compound Represented by Formula (3), Compound Represented by Formula (4)>

The polythiol composition of embodiment B contains at least one compound selected from the group consisting of a compound represented by the following Formula (3) and a compound represented by the following Formula (4).

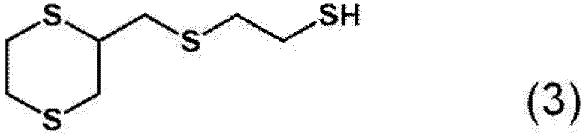

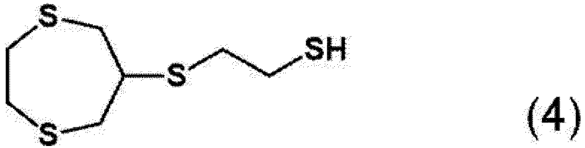

The polythiol composition of embodiment B enables, owing to containing at least one compound selected from the group consisting of a compound represented by Formula (3) and a compound represented by Formula (4), reduction of yellow index and degree of opacity of the resulting resin when the combination of the polythiol compound (A) and the specific sulfur compound is contained in the polymerizable composition.

In the polythiol composition of embodiment B, in a high-performance liquid chromatography measurement, a total peak area of the at least one compound selected from the group consisting of a compound represented by Formula (3) and a compound represented by Formula (4) is 2.10 or less with respect to a total peak area 100 of compounds contained in the polythiol composition.

The total peak area of the at least one compound selected from the group consisting of a compound represented by Formula (3) and a compound represented by Formula (4) being 2.10 or less enables reduction of yellow index and degree of opacity of the resulting resin.

From the same viewpoint as above, the total peak area of the at least one compound selected from the group consisting of a compound represented by Formula (3) and a compound represented by Formula (4) is preferably 2.05 or less, more preferably 1.90 or less, and still more preferably 1.50 or less with respect to the total peak area 100 of the compounds contained in the polythiol composition.

The "total peak area of the at least one compound selected from the group consisting of a compound represented by Formula (3) and a compound represented by Formula (4) with respect to a total peak area 100 of compounds contained in the polythiol composition" means a relative value of the total peak area of the at least one compound selected from the group consisting of a compound represented by Formula (3) and a compound represented by Formula (4) in the case in which the total peak area of the compounds contained in the polythiol composition is taken as 100.

In this regard, for example, when the polythiol composition contains both the compound represented by Formula (3) and the compound represented by Formula (4), the total peak area of the at least one compound selected from the group consisting of a compound represented by Formula (3) and a compound represented by Formula (4) means the total peak area of the compound represented by Formula (3) and the compound represented by Formula (4). When the polythiol composition contains only one of the compound represented by Formula (3) or the compound represented by Formula (4), the total peak area of the at least one compound selected from the group consisting of a compound represented by Formula (3) and a compound represented by Formula (4) means the peak area of the only one of the compounds.

In a high performance liquid chromatography measurement, the total peak area of the at least one compound selected from the group consisting of a compound represented by Formula (3) and a compound represented by Formula (4) is preferably more than 0, more preferably 0.10 or more, and still more preferably 0.30 or more, with respect to the total peak area 100 of the compounds contained in the polythiol composition, from the viewpoint of reducing the burden of work of purifying and removing the at least one compound selected from the group consisting of a compound represented by Formula (3) and a compound represented by Formula (4) from the polythiol composition.

In the case in which the total peak area of the at least one compound selected from the group consisting of a compound represented by Formula (3) and a compound represented by Formula (4) satisfies the range of the lower limit, the rate of thickening can be enhanced, and thus polymerization can be facilitated.

A method of adjusting the total peak area of the at least one compound selected from the group consisting of a compound represented by Formula (3) and a compound represented by Formula (4) with respect to the total peak area 100 of the compounds contained in the polythiol composition is not particularly limited, and the total peak area can be adjusted by, for example, operations such as column purification, washing, extraction, or crystallization.

### <Measurement of Peak Area of Compound Represented by Formula (3) and Compound Represented by Formula (4)>

The total peak area of the at least one compound selected from the group consisting of a compound represented by Formula (3) and a compound represented by Formula (4) with respect to the total peak area 100 of the compounds contained in the polythiol composition can be determined by carrying out a high-performance liquid chromatography (HPLC) measurement based on the following conditions.

The peak area appearing at a retention time of 9.8 to 11.2 minutes may be regarded as the peak area of the compound represented by Formula (3) and the compound represented by Formula (4), and the ratio thereof with respect to the total peak area 100 of the compounds contained in the polythiol composition may be calculated.

### (HPLC conditions)

Column: YMC-Pack ODS-AA-312 (S5 Φ 6 mm × 150 mm)
Mobile phase: acetonitrile / 0.01 mol/L-potassium dihydrogen phosphate aqueous solution = 60/40 (vol/vol)
Column temperature: 40°C
Flow rate: 1.0 ml/min
Detector: UV detector, wavelength 230 nm
Preparation of measurement solution: 160 mg of the polythiol composition is dissolved and mixed with 10 ml of acetonitrile
Injection amount: 2 µL

### <<Polymerizable Composition>>

The polymerizable composition of the present disclosure contains the polythiol composition of the present disclosure and a polyiso(thio)cyanate compound.

### (Polyiso(thio)cyanate Compound)

The polyiso(thio)cyanate compound is not particularly limited as long as the effects of the present disclosure can be exerted, and any conventionally known compound can be used. Any compound having at least two iso(thio)cyanate groups in one molecule may be used without particular limitation, and specific examples thereof include aliphatic polyisocyanate compounds such as tetramethylene diisocyanate, pentamethylene diisocyanate, hexamethylene diisocyanate, heptamethylene diisocyanate, octamethylene diisocyanate, 2,2,4-trimethylhexamethylene diisocyanate, 2,4,4-trimethylhexamethylene diisocyanate, lysine diisocyanate methyl ester, lysine triisocyanate, and xylylene diisocyanate;
alicyclic polyisocyanate compounds such as isophorone diisocyanate, bis(isocyanatomethyl)cyclohexane, bis(isocyanatocyclohexyl)methane, dicyclohexyldimethylmethane diisocyanate, 2,5-bis(isocyanatomethyl)bicyclo-[2.2.1]-heptane, 2,6-bis(isocyanatomethyl)bicyclo-[2.2.1]-heptane, 3,8-bis(isocyanatomethyl)tricyclodecane, 3,9-bis(isocyanatomethyl)tricyclodecane, 4,8-bis(isocyanatomethyl)tricyclodecane, and 4,9-bis(isocyanatomethyl)tricyclodecane;
aromatic polyisocyanate compounds such as tolylene diisocyanate, 4,4'-diphenylmethane diisocyanate, diphenyl sulfide-4,4-diisocyanate, and phenylene diisocyanate;
heterocyclic polyisocyanate compounds such as 2,5-diisocyanatothiophene, 2,5-bis(isocyanatomethyl)thiophene, 2,5-diisocyanatotetrahydrothiophene, 2,5-bis(isocyanatomethyl)tetrahydrothiophene, 3,4-bis(isocyanatomethyl)tetrahydrothiophene, 2,5-diisocyanato-1,4-dithiane, 2,5-bis(isocyanatomethyl)-1,4-dithiane, 4,5-diisocyanato-1,3-dithiolane, and 4,5-bis(isocyanatomethyl)-1,3-dithiolane;
aliphatic polyisothiocyanate compounds such as hexamethylene diisothiocyanate, lysine diisothiocyanate methyl ester, lysine triisothiocyanate, and xylylene diisothiocyanate;
alicyclic polyisothiocyanate compounds such as isophorone diisothiocyanate, bis(isothiocyanatomethyl)cyclohexane, bis(isothiocyanatocyclohexyl)methane, cyclohexane diisothiocyanate, methylcyclohexane diisothiocyanate, 2,5-bis(isothiocyanatomethyl)bicyclo-[2.2.1]-heptane, 2,6-bis(isothiocyanatomethyl)bicyclo-[2.2.1]-heptane, 3,8-bis(isothiocyanatomethyl)tricyclodecane, 3,9-bis(isothiocyanatomethyl)tricyclodecane, 4,8-bis(isothiocyanatomethyl)tricyclodecane, and 4,9-bis(isothiocyanatomethyl)tricyclodecane;
aromatic polyisothiocyanate compounds such as tolylene diisothiocyanate, 4,4-diphenylmethane diisothiocyanate, and diphenyl disulfide-4,4-diisothiocyanate; and
sulfur-containing heterocyclic polyisothiocyanate compounds such as 2,5-diisothiocyanatothiophene, 2,5-bis(isothiocyanatomethyl)thiophene, 2,5-isothiocyanatotetrahydrothiophene, 2,5-bis(isothiocyanatomethyl)tetrahydrothiophene, 3,4-bis(isothiocyanatomethyl)tetrahydrothiophene, 2,5-diisothiocyanato-1,4-dithiane, 2,5-bis(isothiocyanatomethyl)-1,4-dithiane, 4,5-diisothiocyanato-1,3-dithiolane, and 4,5-bis(isothiocyanatomethyl)-1,3-dithiolane. The polyiso(thio)cyanate compound can contain at least one selected from these compounds.

As the polyiso(thio)cyanate compound, a halogen-substituted product such as a chlorine-substituted product or a bromine-substituted product, an alkyl-substituted product, an alkoxy-substituted product, a nitro-substituted product, a prepolymer-type modified product with a polyhydric alcohol, a carbodiimide-modified product, a urea-modified product, a burette-modified product, or a dimerization or trimerization reaction product of these compounds can also be used.

The polyiso(thio)cyanate compound is preferably a polyisocyanate compound, and preferably comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

The mixing ratio of the polythiol composition and the polyiso(thio)cyanate compound is not particularly limited, and for example, the molar ratio of mercapto groups of the polythiol compound contained in the polythiol composition to iso(thio)cyanate groups of the polyiso(thio)cyanate compound (mercapto groups/iso(thio)cyanate groups) is preferably from 0.5 to 3.0, more preferably from 0.6 to 2.0, and still more preferably from 0.8 to 1.3. In the case in which the mixing ratio is within the above range, it tends to be possible to satisfy various performance characteristics such as a refractive index and heat resistance required for a plastic lens or the like in a well-balanced manner.

The polymerizable composition of the present disclosure may contain a polyiso(thio)cyanate composition containing the polyiso(thio)cyanate compound.

Herein, a polyisocyanate composition means a composition containing at least one polyisocyanate compound.

The polyisocyanate composition may contain, as an impurity, a component other than a polyisocyanate compound.

The polyisocyanate composition preferably contains, as a main component, at least one polyisocyanate compound.

The meaning of "contains, as a main component" is as described above.

The polyisocyanate composition preferably contains xylylene diisocyanate.

Hereinafter, a polyisocyanate composition containing xylylene diisocyanate is also referred to as an XDI composition.

The XDI composition preferably contains xylylene diisocyanate as a main component.

The XDI composition preferably contains at least one selected from the group consisting of the following compound (N1), the following compound (N2), and the following compound (N3).

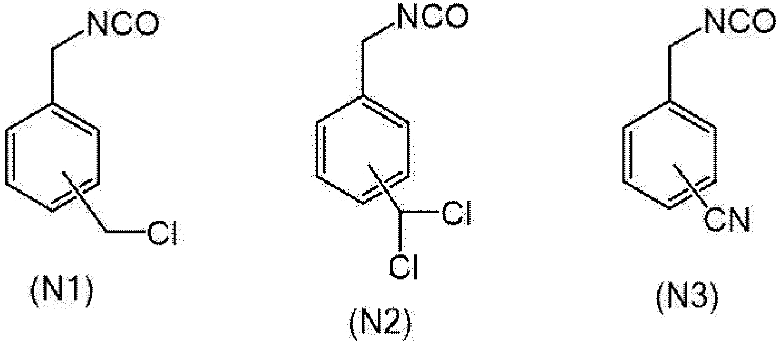

Hereinafter, preferable aspects of the XDI composition are shown from the viewpoints of stability of the polyiso(thio)cyanate composition and more excellent transparency of a resin formed using the polyiso(thio)cyanate composition.

In a case in which the XDI composition contains the compound (N1), a peak area of the compound (N1) is preferably 0.20 ppm or more with respect to a peak area 1 of xylylene diisocyanate in gas chromatography measurement under the following GC condition 1.

### - GC condition 1 -

Packing material; DB-1 (film thickness) 1.5 µm
Column; internal diameter 0.53 mm × length 60 m (manufactured by Agilent Technologies, Inc.)
Oven temperature; increased at 3°C/min from 130°C to 220°C, and increased at 10°C/min to 300°C after reaching 220°C
Split ratio; pulsed splitless method
Inlet temperature; 280°C
Detector temperature; 300°C
Carrier gas; N₂ 158 kPa, H₂ 55 kPa, Air 45 kPa (constant pressure control)
Solvent; chloroform
Sample concentration: 2.0 mass% chloroform solution
Injection amount; 2 µL
Detection method; FID

The peak area of the compound (N1) is more preferably 5.0 ppm or more, still more preferably 50 ppm or more, and still more preferably 100 ppm or more with respect to the peak area 1 of xylylene diisocyanate.

The peak area of the compound (N1) is preferably 4000 ppm or less, more preferably 3000 ppm or less, still more preferably 2000 ppm or less, still more preferably 1500 ppm or less, and still more preferably 1000 ppm or less with respect to the peak area 1 of xylylene diisocyanate.

The peak area of the compound (N1) can be measured in accordance with the method described in paragraph [0377] of Japanese patent No. 6373536.

In a case in which the XDI composition contains the compound (N2), a peak area of the compound (N2) is preferably 0.05 ppm or more with respect to a peak area 1 of xylylene diisocyanate in gas chromatography measurement under the following GC condition 2.

### - GC condition 2 -

Column; HP-50+, internal diameter 0.25 mm × length 30 m × film thickness 0.25 µm (manufactured by Hewlett-Packard Company)
Oven temperature; increased at 10°C/min from 50°C to 280°C, and held for 6 min after reaching 280°C
Split ratio; pulsed splitless method
Inlet temperature; 200°C
Detector temperature; 280°C
Carrier gas; He
Carrier gas flow rate; 1.0 ml/min (constant flow rate control)
Sample concentration: 1.0 mass% dichloromethane solution
Injection amount; 1.0 µL
Detection method; SIM (monitoring ion: m/z 180, 215) (Xylylene diisocyanate (XDI) content ratio)

The peak area of the compound (N2) is more preferably 0.1 ppm or more, still more preferably 0.3 ppm or more, and still more preferably 0.6 ppm or more with respect to the peak area 1 of xylylene diisocyanate.

The peak area of the compound (N2) is preferably 200 ppm or less, more preferably 150 ppm or less, still more preferably 100 ppm or less, still more preferably 80 ppm or less, still more preferably 70 ppm or less, and still more preferably 60 ppm or less with respect to the peak area 1 of xylylene diisocyanate.

The peak area of the compound (N2) can be measured in accordance with the method described in paragraphs [0375] and [0376] of Japanese patent No. 6373536.

In a case in which the XDI composition contains the compound (N3), a peak area of the compound (N3) is preferably 0.10 ppm or more with respect to a peak area 1 of xylylene diisocyanate in gas chromatography measurement under the above GC condition 1.

The peak area of the compound (N3) is more preferably 0.1 ppm or more, still more preferably 3.0 ppm or more, and still more preferably 5.0 ppm or more with respect to the peak area 1 of xylylene diisocyanate.

The peak area of the compound (N3) is preferably 1000 ppm or less, more preferably 500 ppm or less, still more preferably 300 ppm or less, still more preferably 100 ppm or less, and still more preferably 75 ppm or less with respect to the peak area 1 of xylylene diisocyanate.

The peak area of the compound (N3) can be measured in accordance with the method described in paragraph [0377] of Japanese patent No. 6373536.

The acid content in the XDI composition is preferably 3000 ppm or less, more preferably 2000 ppm or less, still more preferably 1000 ppm or less, still more preferably 100 ppm or less, still more preferably 50 ppm or less, still more preferably 30 ppm or less, and still more preferably less than 15 ppm.

The lower limit value of the acid content in the XDI composition is not particularly limited, and the lower limit value is, for example, 1 ppm.

The acid content in the XDI composition can be measured in accordance with the method described in paragraph [0091] of WO 2021/256417.

Further, the XDI composition may contain a stabilizing agent.

The polymerizable composition of the present disclosure may contain other components other than the polythiol compound and the polyiso(thio)cyanate compound for the purpose of improving various physical properties and operability of the resin, polymerization reactivity of the polymerizable composition, and the like.

Examples of the other components include a polymerization catalyst, an internal mold release agent, a resin modifier, a chain extender, a crosslinking agent, a radical scavenger, a light stabilizer, an ultraviolet absorber, an antioxidant, an oil soluble dye, a filler, an adhesion improver, an antibacterial agent, an antistatic agent, a dye, a fluorescent brightener, a fluorescent pigment, and a blue ink agent such as an inorganic pigment.

Examples of the polymerization catalyst include a tertiary amine compound, an inorganic acid salt or organic acid salt thereof, a metal compound, a quaternary ammonium salt, and an organic sulfonic acid.

As the internal mold release agent, an acidic phosphate ester can be used. Examples of the acidic phosphate ester include a phosphoric acid monoester and a phosphoric acid diester, which can be used singly or in a mixture of two or more kinds thereof.

Examples of the resin modifier include an episulfide compound, an alcohol compound, an amine compound, an epoxy compound, an organic acid and an anhydride thereof, and an olefin compound including a (meth)acrylate compound.

The polymerizable composition of the present disclosure can be obtained by mixing the above components.

### <<Molded Body>>

The molded body of the present disclosure contains the resin of the present disclosure.

The resin of the present disclosure contains a cured product of the polymerizable composition of the present disclosure.

The method of producing the molded body of the present disclosure is not particularly limited, and examples of a preferred production method include cast polymerization. First, a polymerizable composition is poured between molding molds held with a gasket, tape, or the like. At this time, depending on physical properties required for a plastic lens to be obtained, it is often preferable to perform defoaming treatment under reduced pressure, filtration treatment such as filtration under pressure or reduced pressure, or the like if necessary.

The polymerization conditions are not limited because the conditions greatly vary depending on the composition of the polymerizable composition, the type and amount of the catalyst to be used, the shape of the mold, and the like. For example, the polymerization is performed at a temperature of from -50°C to 150°C over from 1 hour to 50 hours. In some cases, it is preferable to cure the composition in from 1 hour to 48 hours while the temperature is maintained or gradually raised in a temperature range of 10°C to 150°C.

The molded body may be subjected to a treatment such as annealing if necessary. The treatment such as annealing is usually performed at from 50°C to 150°C, preferably at from 90°C to 140°C, and more preferably at from 100°C to 130°C.

### [Applications]

The resin to be obtained from the polymerizable composition of the present disclosure can be used as a material for producing molded bodies of various shapes by changing the type of mold during cast polymerization.

### <<Optical Material>>

The optical material of the present disclosure contains the resin of the present disclosure.

From a molded body obtained from the polymerizable composition of the present disclosure, it is possible to obtain a material having a reduced yellow index without impairing transparency. From a molded body obtained from the polymerizable composition containing the polythiol composition of the first embodiment, it is also possible to obtain a material having excellent degree of opacity.

Thus, such materials can be used for various optical materials such as plastic lenses.

### <<Lens>>

The lens of the present disclosure contains the resin of the present disclosure.

As the optical material, a lens is particularly suitable.

Examples of the lens include a plastic spectacle lens and a plastic polarizing lens.

### [Plastic Spectacle Lens]

The plastic spectacle lens including a lens substrate formed of the molded body of the present disclosure may be provided with a coating layer on one side or both sides if necessary.

The plastic spectacle lens of the present disclosure comprises a lens substrate including a cured product of the polymerizable composition described above, and a coating layer.

Specific examples of the coating layer include a primer layer, a hard coat layer, an antireflection layer, an antifogging coat layer, an antifouling layer, and a water-repellent layer. These coating layers each can be used singly, or a plurality of coating layers can be layered for use. In the case in which the coating layer is applied to both sides, the same coating layer or a different coating layer may be applied to each side.

In each of these coating layers, known additives such as an infrared absorber for the purpose of protecting eyes from infrared rays, a light stabilizer, or an antioxidant for the purpose of improving the weather resistance of the lens, a photochromic compound, a dye and a pigment for the purpose of improving the fashionability of the lens, and additionally, an antistatic agent and the like for the purpose of improving the performance of the lens may be used in combination.

For the layer to be coated by coating, various leveling agents for the purpose of improving coatability may be used.

An anti-fog layer, an anti-contamination layer, or a water-repellent layer may be formed on the antireflection layer if necessary.

Although the embodiments of the present disclosure have been described hereinabove, these are exemplary for the present disclosure, and various configurations other than those described above can be adopted as long as the effects of the present disclosure are not impaired.

### EXAMPLES

Hereinafter, the present disclosure will be described in detail with reference to Examples. The present disclosure is not limited to the description of these Examples in any way. Unless otherwise specified, "parts" are on a mass basis.

### <Evaluation Methods>

In the Examples, methods for evaluating each of the physical properties of plastic lenses are as follows.

### • Yellow Index (also referred to as YI)

A resin was prepared as a circular plastic flat plate having a thickness of 9 mm and a diameter of 75 mm, and the YI value was determined using a spectrophotometer CM-5 manufactured by Konica Minolta, Inc.

There is a correlation between the YI value and the yellow index, in which a smaller YI value leads to a lower yellow index of the plastic flat plate and a larger YI value leads to a higher yellow index.

### • Degree of opacity

A resin was prepared as a circular plastic flat plate having a thickness of 9 mm and a diameter of 75 mm, and light from a light source (LUMINAR ACE LA-150 A manufactured by HAYASHI-REPIC CO., LTD.) was allowed to pass through the flat plate from a side of the flat plate. An image of light from the front of the flat plate was captured in an image processing apparatus (manufactured by Ube Information Systems, Inc.), and contrasting density processing was performed on the captured image. The degree of density of the processed image was quantified on a pixel to pixel basis, the average value of the numerical values of the degree of density of each pixel was obtained, and the degree of opacity of the flat plate was determined.

With a smaller degree of opacity, the degree of impairing the transparency of the resin (here, the flat plate) is smaller (i.e., the resin has excellent transparency).

### <Production of Polythiol Composition (A)>

Into a reactor were charged 124.6 parts by mass of 2-mercaptoethanol and 18.3 parts by mass of degassed water. At from 12°C to 35°C, 101.5 parts by mass of a 32 mass% aqueous solution of sodium hydroxide were added dropwise over 40 minutes, then 73.6 parts by mass of epichlorohydrin were added dropwise over 4.5 hours at from 29°C to 36°C, and stirring was continued for 40 minutes. The NMR data confirmed that 1,3 bis(2-hydroxyethylthio)-2-propanol was generated.

331.5 parts by mass of 35.5% hydrochloric acid were charged, then 183.8 parts by mass of thiourea having a purity of 99.90% were charged, and a reaction of thiuronium salt production was performed by stirring the resulting mixture at 110°C under reflux for 3 hours. After cooling to 45°C, 320.5 parts by mass of toluene were added, the mixture was cooled to 31°C, and 243.1 parts by mass of a 25% aqueous ammonia solution were charged at from 31°C to 41°C over 44 minutes. The mixture was subjected to a hydrolysis reaction by stirring at from 54°C to 62°C for 3 hours to obtain a toluene solution of a polythiol containing 4-mercaptomethyl-1,8-dimercapto-3,6-dithiaoctane as a main component. To this toluene solution, 162.8 parts by mass of 35.5% hydrochloric acid were added, and the mixture was acid-washed at from 35°C to 43°C for 1 hour. 174.1 parts by mass of degassed water were added, and the mixture was washed twice at from 35°C to 45°C for 30 minutes. 162.1 parts by mass of 0.1% ammonia water were added, and the mixture was washed for 30 minutes. 174.2 parts by mass of degassed water were added, and the mixture was washed twice at from 35°C to 45°C for 30 minutes. Toluene and a trace amount of moisture were removed under reduced pressure with heating, and then filtration was carried out under reduced pressure with a 1.2 µm PTFE-type membrane filter to obtain 205.0 parts by mass of a polythiol composition (A) containing 4-mercaptomethyl-1,8-dimercapto-3,6-dithiaoctane as the polythiol compound (A) as a main component.

### <Production of Polythiol Composition (A1) Containing Compound Represented by Formula (1) and Compound Represented by Formula (2)>

Into a reactor were added 200.0 parts by mass of a polythiol composition containing 4-mercaptomethyl-1,8-dimercapto-3,6-dithiaoctane as a main component, 4.0 parts by mass of diethylenetriamine, 400 ml of toluene, and 100 ml of water, followed by heating and stirring at from 80 to 85°C for 5 hours. After being left to stand to be separated into two layers, the lower aqueous layer was discharged, and to the upper layer (toluene layer) remaining in the reactor was added 200 parts by mass of 35% hydrochloric acid, followed by carrying out twice acid washing at from 35 to 40°C for 1 hour. After being left to stand to be separated into two layers, the lower aqueous layer was discharged, and to the toluene layer was added 200.0 parts by mass of degassed water, followed by carrying out washing at from 35 to 40°C for 30 minutes. After being left to stand to be separated into two layers, the lower aqueous layer was discharged, and to the toluene layer was added 200.0 parts by mass of 0.1% ammonia water, followed by carrying out washing for 30 minutes. After being left to stand to be separated into two layers, the lower aqueous layer was discharged, and to the toluene layer was added 200.0 parts by mass of degassed water, followed by carrying out twice washing for 30 minutes. Toluene and a trace amount of moisture were removed under reduced pressure with heating, and then filtration was carried out under reduced pressure with a 1.2 µm PTFE-type membrane filter to obtain 188.7 parts by mass of a polythiol composition (A1) containing 4-mercaptomethyl-1,8-dimercapto-3,6-dithiaoctane as a polythiol compound as a main component.

A compound represented by Formula (1) and a compound represented by Formula (2) were contained in total in an amount of 1.50 with respect to the total peak area 100 of the compounds contained in the obtained polythiol composition (A1).

### <Production of Polythiol Composition (A2) Containing Compound Represented by Formula (3) and Compound Represented by Formula (4)>

Into a reactor were charged 152.19 parts by mass of 2-mercaptoethanol and 23.1 parts by mass of degassed water. At from 15°C to 30°C, 128.32 parts by mass of a 30.3 mass% aqueous solution of sodium hydroxide were added dropwise over 30 minutes, then 89.99 parts by mass of epichlorohydrin were added dropwise over 3 hours at from 27°C to 30°C, and stirring was continued for 1 hour.

405.1 parts by mass of 35% hydrochloric acid were charged, then 224.3 parts by mass of thiourea having a purity of 99.4% were charged, and a reaction of thiuronium salt production was performed by stirring the resulting mixture at 110°C under reflux for 3 hours. After cooling to 40°C, 395.0 parts by mass of toluene were added, the mixture was cooled to 34°C, and 293.3 parts by mass of a 25.4 mass% aqueous ammonia solution were charged at from 34°C to 40°C over 25 minutes. The mixture was subjected to a hydrolysis reaction by stirring at from 60°C to 62°C for 3 hours to obtain a toluene solution of a polythiol composition containing 4-mercaptomethyl-1,8-dimercapto-3,6-dithiaoctane as a main component. To this toluene solution, 180.0 parts by mass of 35% hydrochloric acid were added, and the mixture was acid-washed twice at from 75°C to 80°C for 3 hours. 200.0 parts by mass of degassed water were added, and the mixture was washed at from 35°C to 45°C for 30 minutes. 200.0 parts by mass of 0.1% ammonia water were added, and the mixture was washed for 30 minutes. 200.0 parts by mass of degassed water were added, and the mixture was washed twice for 30 minutes. Toluene and a trace amount of moisture were removed under reduced pressure with heating, and then filtration was carried out under reduced pressure with a 1.2 µm PTFE-type membrane filter to obtain 247.1 parts by mass of a polythiol composition (A2) containing 4-mercaptomethyl-1,8-dimercapto-3,6-dithiaoctane as the polythiol compound (A) as a main component.

A compound represented by Formula (3) was contained in an amount of 1.31 and a compound represented by Formula (4) was contained in an amount of 1.50 with respect to the total peak area 100 of the compounds contained in the obtained polythiol composition (A2).

### [Example 1 to Example 3]

The polythiol composition (A) and the polythiol composition (A1) obtained as described above were mixed to obtain a polythiol composition.

At this time, the respective compositions were mixed while the mixing ratio was changed such that the total peak area of the compound represented by Formula (1) and the compound represented by Formula (2) with respect to the total peak area 100 of the compounds contained in the polythiol composition was the value described in Table 1.

### [Comparative Example 1]

The polythiol composition (A1) was used as the polythiol composition.

### <Measurement of Proportion (area%) of Compound Represented by Formula (1) and Compound Represented by Formula (2)>

The proportion (area%) of the compound represented by Formula (1) and the compound represented by Formula (2) by HPLC was measured by the method described in the section of <Measurement of Peak Area of Compound Represented by Formula (1) and Compound Represented by Formula (2)> described above.

The results are shown in Table 1.

### <Production of Plastic Lens>

### [Production Example 1]

52 parts by mass of m-xylylene diisocyanate, 0.01 parts by mass of dibutyltin dichloride as a curing catalyst, 0.10 parts by mass of ZELEC UN (trade name, a product manufactured by Stepan Company; acidic phosphate ester), and 1.5 parts by mass of VIOSORB 583 (manufactured by KYODO CHEMICAL CO., LTD.; ultraviolet absorber) were mixed and dissolved at 20°C. 48 parts by mass of the polythiol composition of Example 1 were charged and mixed to obtain a mixed homogeneous liquid. After this homogeneous liquid was degassed at 600 Pa for 1 hour and filtered with a 1 µm TEFLON (R) filter, the filtered liquid was poured into a mold die composed of a glass mold and tape. The mold die was placed into an oven, gradually heated up from 10°C to 120°C, and polymerization was performed for 38 hours. After the polymerization was finished, the mold die was taken out from the oven to obtain a resin by releasing from the mold die. The obtained resin was further annealed at 120°C for 1 hour to produce a plastic lens. Physical properties were each determined based on each of the methods of evaluating physical properties of the plastic lens described above.

### [Production Example 2]

A plastic lens was produced by the same method as that described in Production Example 1 except that 48 parts by mass of the polythiol composition of Example 1 were replaced by 48 parts by mass of the polythiol composition of Example 2 in Production Example 1. Physical properties were each determined based on each of the methods of evaluating physical properties of the plastic lens described above.

### [Production Example 3]

A plastic lens was produced by the same method as that described in Production Example 1 except that 48 parts by mass of the polythiol composition of Example 1 were replaced by 48 parts by mass of the polythiol composition of Example 3 in Production Example 1. Physical properties were each determined based on each of the methods of evaluating physical properties of the plastic lens described above.

### [Production Example 4]

A plastic lens was produced by the same method as that described in Production Example 1 except that 48 parts by mass of the polythiol composition of Example 1 were replaced by 48 parts by mass of the polythiol composition of Comparative Example 1 in Production Example 1. Physical properties were each determined based on each of the methods of evaluating physical properties of the plastic lens described above.

Physical properties of the plastic lenses of Production Examples 1 to 4 (that is, Examples 1 to 3 and Comparative Example 1) are shown in Table 1.

**[Table 1]**

| | | Example 1 | Example 2 | Example 3 | Comparative Example 1 |
|---|---|---|---|---|---|
| Polythiol composition | Proportion (area%) of total peak area of compound represented by Formula (1) and compound represented by Formula (2) with respect to total peak area 100 of compounds contained in polythiol composition | 0.15 | 0.40 | 0.80 | 1.50 |
| Plastic lens | YI | 4.0 | 4.1 | 4.1 | 4.5 |
| | Degree of opacity | 22 | 25 | 27 | 41 |

As shown in Table 1, Examples 1 to 3, which contained: a polythiol compound (A); and at least one compound selected from the group consisting of a compound represented by Formula (1) and a compound represented by Formula (2), wherein, in a high-performance liquid chromatography measurement, the total peak area of the at least one compound selected from the group consisting of a compound represented by Formula (1) and a compound represented by Formula (2) was 1.00 or less with respect to the total peak area 100 of compounds contained in the polythiol composition, were excellent in YI and degree of opacity.

In contrast, Comparative Example 1, wherein, the total peak area of the at least one compound selected from the group consisting of a compound represented by Formula (1) and a compound represented by Formula (2) was not 1.00 or less, was inferior in YI and degree of opacity.

### [Reference Example 1 to Reference Example 3]

The polythiol composition (A) and the polythiol composition (A2) obtained as described above were mixed to obtain a polythiol composition.

At this time, the respective compositions were mixed while the mixing ratio was changed such that the total peak area of the compound represented by Formula (3) and the compound represented by Formula (4) with respect to the total peak area 100 of the compounds contained in the polythiol composition was the value described in Table 2.

### [Comparative Example 2]

The polythiol composition (A2) was used as the polythiol composition.

### <Measurement of Proportion (area%) of Compound Represented by Formula (3) and Compound Represented by Formula (4)>

The proportion (area%) of the compound represented by Formula (3) and the compound represented by Formula (4) by HPLC was measured by the method described in the section of <Measurement of Peak Area of Compound Represented by Formula (3) and Compound Represented by Formula (4)> described above.

The results are shown in Table 2.

### <Production of Plastic Lens>

### [Production Example 5]

52 parts by mass of m-xylylene diisocyanate, 0.01 parts by mass of dibutyltin dichloride as a curing catalyst, 0.10 parts by mass of ZELEC UN (trade name, a product manufactured by Stepan Company; acidic phosphate ester), and 1.5 parts by mass of VIOSORB 583 (manufactured by KYODO CHEMICAL CO., LTD.; ultraviolet absorber) were mixed and dissolved at 20°C. 48 parts by mass of the polythiol composition of Reference Example 1 were charged and mixed to obtain a mixed homogeneous liquid. After this homogeneous liquid was degassed at 600 Pa for 1 hour and filtered with a 1 µm TEFLON (R) filter, the filtered liquid was poured into a mold die composed of a glass mold and tape. The mold die was placed into an oven, gradually heated up from 10°C to 120°C, and polymerization was performed for 38 hours. After the polymerization was finished, the mold die was taken out from the oven to obtain a resin by releasing from the mold die. The obtained resin was further annealed at 120°C for 1 hour to produce a plastic lens. Physical properties were each determined based on each of the methods of evaluating physical properties of the plastic lens described above.

### [Production Example 6]

A plastic lens was produced by the same method as that described in Production Example 1 except that 48 parts by mass of the polythiol composition of Reference Example 1 were replaced by 48 parts by mass of the polythiol composition of Reference Example 2 in Production Example 5. Physical properties were each determined based on each of the methods of evaluating physical properties of the plastic lens described above.

### [Production Example 7]

A plastic lens was produced by the same method as that described in Production Example 1 except that 48 parts by mass of the polythiol composition of Reference Example 1 were replaced by 48 parts by mass of the polythiol composition of Reference Example 3 in Production Example 5. Physical properties were each determined based on each of the methods of evaluating physical properties of the plastic lens described above.

### [Production Example 8]

A plastic lens was produced by the same method as that described in Production Example 1 except that 48 parts by mass of the polythiol composition of Reference Example 1 were replaced by 48 parts by mass of the polythiol composition of Comparative Example 2 in Production Example 5. Physical properties were each determined based on each of the methods of evaluating physical properties of the plastic lens described above.

Physical properties of the plastic lenses of Production Examples 5 to 8 (that is, Reference Examples 1 to 3 and Comparative Example 2) are shown in Table 2.

**[Table 2]**

| | | Reference Example 1 | Reference Example 2 | Reference Example 3 | Comparative Example 2 |
|---|---|---|---|---|---|
| Polythiol composition | Proportion (area%) of peak area of compound represented by Formula (3) with respect to total peak area 100 of compounds contained in polythiol composition | 0.18 | 0.58 | 0.95 | 1.31 |
| | Proportion (area%) of peak area of compound represented by Formula (4) with respect to total peak area 100 of compounds contained in polythiol composition | 0.14 | 0.61 | 1.05 | 1.50 |
| | Proportion (area%) of total peak area of compound represented by Formula (3) and compound represented by Formula (4) with respect to total peak area 100 of compounds contained in polythiol composition | 0.32 | 1.19 | 2.00 | 2.81 |
| Plastic lens | YI | 3.9 | 4.1 | 4.3 | 5.4 |

As shown in Table 2, Reference Examples 1 to 3, which contained: a polythiol compound (A); and at least one compound selected from the group consisting of a compound represented by Formula (3) and a compound represented by Formula (4), wherein, in a high-performance liquid chromatography measurement, the total peak area of the at least one compound selected from the group consisting of a compound represented by Formula (3) and a compound represented by Formula (4) was 2.10 or less with respect to the total peak area 100 of compounds contained in the polythiol composition, were excellent in YI.

In contrast, Comparative Example 2, wherein, the total peak area of the at least one compound selected from the group consisting of a compound represented by Formula (3) and a compound represented by Formula (4) was not 2.10 or less, was inferior in YI.

### [Examples 1X, 2X, and 3X, and Reference Examples 1X, 2X, and 3X]

When, as each of Examples 1X, 2X, and 3X, and Reference Examples 1X, 2X, and 3X, a molded body was produced by performing the same procedure as that in each of Examples 1, 2, and 3, and Reference Examples 1, 2, and 3 except for the following modifications, the same results as the results (Tables 1 and 2) of each of Examples 1, 2, and 3, and Reference Examples 1, 2, and 3 were obtained.

### - Modifications from Each of Examples 1, 2, and 3, and Reference Examples 1, 2, and 3 -

In each of Examples 1, 2, and 3, and Reference Examples 1, 2, and 3, m-xylylene diisocyanate (52 parts by mass) was used when producing the molded body. Meanwhile, in each of Examples 1X, 2X, and 3X, and Reference Examples 1X, 2X, and 3X, the XDI (52 parts by mass) was changed to an XDI composition X1 (in an amount corresponding to 52 parts by mass of m-xylylene diisocyanate) as the XDI composition described above.

The XDI composition X1 was produced by adding a very small amount of the compound (N1), a very small amount of the compound (N2), and a very small amount of the compound (N3) to XDI as a main component, and mixing them.

Gas chromatography measurement was performed for the XDI composition X1 under each of the GC conditions 1 and 2 described above, and as a result, it was found that:
the peak area of the compound (N1) was 0.20 ppm or more (specifically, 600 ppm) with respect to the peak area 1 of xylylene diisocyanate,
the peak area of the compound (N2) was 0.05 ppm or more (specifically, 18 ppm) with respect to the peak area 1 of xylylene diisocyanate, and
the peak area of the compound (N3) was 0.10 ppm or more (specifically, 100 ppm) with respect to the peak area 1 of xylylene diisocyanate.

The disclosure of Japanese Patent Application No. 2022-146425 filed on September 14, 2022 is incorporated herein by reference in its entirety.

All documents, patent applications, and technical standards described herein are incorporated herein by reference to the same extent as if each document, patent application, and technical standard were specifically and individually indicated to be incorporated by reference.

## Claims

1. A polythiol composition, comprising:
a polythiol compound (A); and
at least one compound selected from the group consisting of a compound represented by the following Formula (1) and a compound represented by the following Formula (2): 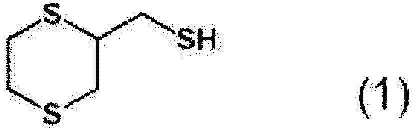 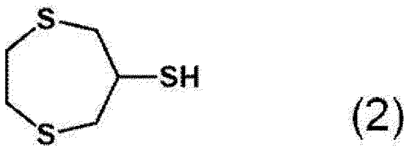
wherein, in a high-performance liquid chromatography measurement, a total peak area of the at least one compound selected from the group consisting of a compound represented by Formula (1) and a compound represented by Formula (2) is 1.00 or less with respect to a total peak area 100 of compounds contained in the polythiol composition.

2. The polythiol composition according to claim 1, wherein the polythiol compound (A) comprises a polythiol compound obtained from 2-mercaptoethanol and thiourea as raw materials.

3. The polythiol composition according to claim 1 or 2, wherein the polythiol compound (A) comprises 4-mercaptomethyl-1,8-dimercapto-3,6-dithiaoctane or comprises a mixture of 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane.

4. A polymerizable composition, comprising:
the polythiol composition according to claim 1 or 2; and
a polyiso(thio)cyanate compound.

5. The polymerizable composition according to claim 4, wherein the polyiso(thio)cyanate compound comprises at least one selected from the group consisting of pentamethylene diisocyanate, hexamethylene diisocyanate, xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, and phenylene diisocyanate.

6. The polymerizable composition according to claim 4, comprising a polyiso(thio)cyanate composition comprising the polyiso(thio)cyanate compound, wherein the polyiso(thio)cyanate composition comprises: xylylene diisocyanate; and at least one selected from the group consisting of the following compound (N1), the following compound (N2), and the following compound (N3): 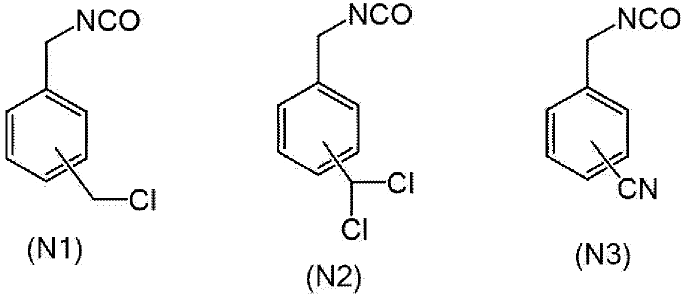 wherein:
in a case in which the polyiso(thio)cyanate composition comprises the compound (N1), a peak area of the compound (N1) is 0.20 ppm or more with respect to a peak area 100 of xylylene diisocyanate in a high-performance liquid chromatography measurement,
in a case in which the polyiso(thio)cyanate composition comprises the compound (N2), a peak area of the compound (N2) is 0.05 ppm or more with respect to a peak area 100 of xylylene diisocyanate in a high-performance liquid chromatography measurement, and
in a case in which the polyiso(thio)cyanate composition comprises the compound (N3), a peak area of the compound (N3) is 0.10 ppm or more with respect to a peak area 100 of xylylene diisocyanate in a high-performance liquid chromatography measurement.

7. A resin, comprising a cured product of the polymerizable composition according to claim 4.

8. A molded body, comprising the resin according to claim 7.

9. An optical material, comprising the resin according to claim 7.

10. A lens, comprising the resin according to claim 7.
